# EUROPEAN PATENT APPLICATION

(11) **EP 2 537 941 A1**
(43) Date of publication of application: **26.12.2012**
(21) Application number: 11739346.2
(22) Date of filing: 30.03.2011
(51) Int. Cl.: C12Q 1/68, C12N 15/11

(54) **METHODS AND NUCLEOTIDE FRAGMENTS OF PREDICTING THE ABILITY OF TUMOR INVASION AND METASTASIS IN VITRO**

(30) Priority: 03.02.2010 CN 201010111138
(71) Applicant: Beijing Institute for Cancer Research, Beijing 100036 (CN)
(72) Inventor: DENG, Dajun, Beijing 100036 (CN); LIU, Zhaojun, Beijing 100036 (CN)
(74) Representative: Ilgart, Jean-Christophe
(86) International application number: PCT/CN2011/000533
(87) International publication number: WO 2011/095067

(57) **Abstract**

The present invention discloses a method for predicting the ability of tumor invasion and metastasis in vitro, including detecting methylation of the cytosine in 5' CpG island of DNA sequence of serum response factor. In addition, the present invention also provides four artificial nucleotide fragments for detection of methylation of the cytosine in CpG island of the nucleotide sequence of serum response factor.

SEQUENCE LISTING

<110>  Beijing Institute for Cancer Research

<120>  METHODS AND NUCLEOTIDE FRAGMENTS OF PREDICTING THE ABILITY OF
       TUMOR INVASION AND METASTASIS IN VITRO

<130>  SR53113 (EP)/SG

<140>  EP11739346
<141>  2011-03-30

<160>  21

<170>  PatentIn version 3.5

<210>  1
<211>  647
<212>  DNA
<213>  Unknown

<220>
<223>  Artificial Sequence


<220>
<221>  misc_feature
<222>  (1)..(647)
<223>  DNA sequence of the SENSE strand of SRF 5′ CpG island in which
       all cytosine residues in CpG sites are methylated and all
       cytosine residues in non-CpG sites are chemically converted to
       uracil residues (n)

<400>  1
gnnanagggg naggaaagtg agggnnnagg tcggnncggg cgtgnagggg nnncgggttc      60

gnagcggcgg ncgcggnagc gatagcggna ntagnagnag cgggagtgnc gggttgagnc     120

gggaagncga tggcggcggn tgcggcggnt ncgattnntc gntgantgnn cgtncgnnnt     180

nntgnatcga gcgnnatgtt ancgannnaa gntggggncg cggcggntnt gggncggggn     240

tcggnnntgg ggggnagnnt gaancggann ncgacggggc ggncgggcgg cggcggcggg     300

anacgcgggg ntaacggggg ncgggtnnnc gggaatggcg cggggntcgg gnncggncgn     360

ntggagcggg aggntgcggn agcggcggna annannncgg cgnnnancgc gggggnnntn     420

tanagcggna gcgagggcga ntcggagtcg ggcgaggagg aggagntggg cgncgagcgg     480

cgcggnntga agcggagnnt gagcgagatg gagatcggta tggtggtcgg tgggnncgag     540

gcgtcggnag cggnnancgg gggntacggg ncggtgagcg gcgcggtgag cggggnnaag     600

ncgggtaaga aganncgggg ncgcgtgaag atnaagatgg agttnat                   647


<210>  2
<211>  647
<212>  DNA
<213>  Unknown

<220>
<223>  Artificial Sequence

<220>
<221> misc_feature
<222> (1)..(647)
<223> DNA sequence of the SENSE strand of SRF 5'CpG island in which all
      cytosine residues are chemically converted to uracil residues (n)

<400> 2
gnnanagggg naggaaagtg agggnnnagg tnggnnnggg ngtgnagggg nnnngggttn      60

gnagnggngg nngnggnagn gatagnggna ntagnagnag ngggagtgnn gggttgagnn     120

gggaagnnga tggnggnggn tgnggnggnt nngattnntn gntgantgnn ngtnngnnnt     180

nntgnatnga gngnnatgtt anngannnaa gntggggnng nggnggntnt gggnnggggn     240

tnggnnntgg ggggnagnnt gaannggann nnganggggn ggnngggngg nggnggnggg     300

anangngggg ntaanggggg nngggtnnnn gggaatggng nggggntngg gnnnggnngn     360

ntggagnggg aggntgnggn agnggnggna annannnngg ngnnnanngn gggggnnntn     420

tanagnggna gngagggnga ntnggagtng ggngaggagg aggagntggg ngnngagngg     480

ngnggnntga agnggagnnt gagngagatg gagatnggta tggtggtngg tgggnnngag     540

gngtnggnag nggnnanngg gggntanggg nnggtgagng gngnggtgag nggggnnaag     600

nngggtaaga agannngggg nngngtgaag atnaagatgg agttnat                   647

<210> 3
<211> 647
<212> DNA
<213> Unknown

<220>
<223> Artificial Sequence

<220>
<221> misc_feature
<222> (1)..(647)
<223> DNA sequence of the ANTISENSE strand of SRF 5' CpG island in
      which all cytosine residues in CpG sites are methylated and all
      cytosine residues in non-CpG sites are chemically converted to
      uracil residues (n)

<400> 3
atgaantnna tnttgatntt nacgcggnnn cgggtnttnt tanncggntt ggnnncgntn      60

ancgcgncgn tnancggnnc gtagnnnncg gtggncgntg ncgacgnntc gggnnnancg     120

annannatan cgatntnnat ntcgntnagg ntncgnttna ggncgcgncg ntcggcgnnn     180

agntnntnnt nntcgnncga ntncgagtcg nnntcgntgn cgntgtagag ggnnnncgcg     240

gtgggcgncg gggtggttgn cgncgntgnc gnagnntnnc gntnnaggcg gncgggnncg     300

agnnncgcgn nattnncggg ganncggnnn ncgttagnnn cgcgtgtnnc gncgncgncg     360

nncggncgnn ncgtcggggt ncggttnagg ntgnnnnnna gggncgagnn ncggnnnaga     420

gncgncgcgg nnnnagnttg ggtcggtaan atggcgntcg atgnaggagg gcggacgggn     480

agtnagcgag gaatcggagn cgncgnagnc gncgnnatcg gnttnncggn tnaanncggn      540

antnncgntg ntgntagtgn cgntatcgnt gncgcggncg ncgntgcgaa nncggggnnn      600

ntgnacgnnc gggncgannt gggnnntnan tttnntgnnn ntgtggn                    647

<210>  4
<211>  647
<212>  DNA
<213>  Unknown

<220>
<223>  Artificial Sequence

<220>
<221>  misc_feature
<222>  (1)..(647)
<223>  DNA sequence of the ANTISENSE strand of SRF 5' CpG island in
       which all cytosine residues are chemically converted to uracil
       residues (n)

<400>  4
atgaantnna tnttgatntt nangnggnnn ngggtnttnt tannnggntt ggnnnngntn       60

anngngnngn tnannggnnn gtagnnnnng gtggnngntg nngangnntn gggnnnanng      120

annannatan ngatntnnat ntngntnagg ntnngnttna ggnngngnng ntnggngnnn      180

agntnntnnt nntngnnnga ntnngagtng nnntngntgn ngntgtagag ggnnnnngng      240

gtgggngnng gggtggttgn ngnngntgnn gnagnntnnn gntnnaggng gnngggnnng      300

agnnnngngn nattnnnggg gannnggnnn nngttagnnn ngngtgtnnn gnngnngnng      360

nnnggnngnn nngtnggggt nnggttnagg ntgnnnnnna gggnngagnn nnggnnnaga      420

gnngnngngg nnnnagnttg ggtnggtaan atggngntng atgnaggagg gnggangggn      480

agtnagngag gaatnggagn ngnngnagnn gnngnnatng gnttnnnggn tnaannnggn      540

antnnngntg ntgntagtgn ngntatngnt gnngnggnng nngntgngaa nnnggggnnn      600

ntgnangnnn gggnngannt gggnnntnan tttnntgnnn ntgtggn                    647

<210>  5
<211>  76
<212>  DNA
<213>  Unknown

<220>
<223>  Artificial Sequence

<220>
<221>  misc_feature
<222>  (1)..(76)
<223>  Artificial nucleotide oligomer in which n represents an uracil
       residue

<400>  5
cggnantagn agnagcggga gtgncgggtt gagncgggaa gncgatggcg gcggntgcgg       60

cggntncgat tnntcg                                                    76

<210>  6
<211>  76
<212>  DNA
<213>  Unknown

<220>
<223>  Artificial Sequence

<220>
<221>  misc_feature
<222>  (1)..(76)
<223>  Artificial nucleotide oligomer in which n represents an uracil
       residue

<400>  6
nggnantagn agnagnggga gtgnngggtt gagnngggaa gnngatggng gnggntgngg      60

nggntnngat tnntng                                                    76

<210>  7
<211>  76
<212>  DNA
<213>  Unknown

<220>
<223>  Artificial Sequence

<220>
<221>  misc_feature
<222>  (1)..(76)
<223>  Artificial nucleotide oligomer in which n represents an uracil
       residue

<400>  7
cgaggaatcg gagncgncgn agncgncgnn atcggnttnn cggntnaann cggnantnnc      60

gntgntgnta gtgncg                                                    76

<210>  8
<211>  76
<212>  DNA
<213>  Unknown

<220>
<223>  Artificial Sequence

<220>
<221>  misc_feature
<222>  (1)..(76)
<223>  Artificial nucleotide oligomer in which n represents an uracil
       residue

<400>  8
ngaggaatng gagnngnngn agnngnngnn atnggnttnn nggntnaann nggnantnnn      60

gntgntgnta gtgnng                                                    76

<210>  9
<211>  139
<212>  DNA
<213>  Unknown

<220>
<223>  Artificial Sequence


<220>
<221>  misc_feature
<222>  (1)..(139)
<223>  Artificial nucleotide oligomer in which n represents an uracil
       residue

<400>  9
cgggaggntg cggnagcggc ggnaannann ncggcgnnna ncgcgggggn nntntanagc       60

ggnagcgagg gcgantcgga gtcgggcgag gaggaggagn tgggcgncga gcggcgcggn      120

ntgaagcgga gnntgagcg                                                   139


<210>  10
<211>  139
<212>  DNA
<213>  Unknown

<220>
<223>  Artificial Sequence


<220>
<221>  misc_feature
<222>  (1)..(139)
<223>  Artificial nucleotide oligomer in which n represents an uracil
       residue

<400>  10
ngggaggntg nggnagnggn ggnaannann nnggngnnna nngngggggn nntntanagn       60

ggnagngagg gngantngga gtngggngag gaggaggagn tgggngnnga gnggngnggn      120

ntgaagngga gnntgagng                                                   139


<210>  11
<211>  139
<212>  DNA
<213>  Unknown

<220>
<223>  Artificial Sequence


<220>
<221>  misc_feature
<222>  (1)..(139)
<223>  Artificial nucleotide oligomer in which n represents an uracil
       residue

<400>  11
cgntnaggnt ncgnttnagg ncgcgncgnt cggcgnnnag ntnntnntnn tcgnncgant       60

ncgagtcgnn ntcgntgncg ntgtagaggg nnnncgcggt gggcgncggg gtggttgncg 120

ncgntgncgn agnntnncg 139

<210> 12
<211> 139
<212> DNA
<213> Unknown

<220>
<223> Artificial Sequence

<220>
<221> misc_feature
<222> (1)..(139)
<223> Artificial nucleotide oligomer in which n represents an uracil
      residue

<400> 12
ngntnaggnt nngnttnagg nngngnngnt nggngnnnag ntnntnntnn tngnnngant 60

nngagtngnn ntngntgnng ntgtagaggg nnnnngnggt gggngnnggg gtggttgnng 120

nngntgnngn agnntnnng 139

<210> 13
<211> 647
<212> DNA
<213> Unknown

<220>
<223> Artificial Sequence

<220>
<221> misc_feature
<222> (1)..(647)
<223> 5' fragment of the SRF gene

<400> 13
gccacagggg caggaaagtg agggcccagg tcggcccggg cgtgcagggg ccccgggttc 60

gcagcggcgg ccgcggcagc gatagcggca ctagcagcag cgggagtgcc gggttgagcc 120

gggaagccga tggcggcggc tgcggcggct ccgattcctc gctgactgcc cgtccgccct 180

cctgcatcga gcgccatgtt accgacccaa gctggggccg cggcggctct gggccggggc 240

tcggccctgg ggggcagcct gaaccggacc ccgacggggc ggccgggcgg cggcggcggg 300

acacgcgggg ctaacggggg ccgggtcccc gggaatggcg cggggctcgg gcccggccgc 360

ctggagcggg aggctgcggc agcggcggca accaccccgg cgcccaccgc gggggccctc 420

tacagcggca gcgagggcga ctcggagtcg ggcgaggagg aggagctggg cgccgagcgg 480

cgcggcctga agcggagcct gagcgagatg gagatcggta tggtggtcgg tgggcccgag 540

gcgtcggcag cggccaccgg gggctacggg ccggtgagcg gcgcggtgag cggggccaag 600

ccgggtaaga agacccgggg ccgcgtgaag atcaagatgg agttcat 647

<210>  14
<211>  22
<212>  DNA
<213>  Unknown

<220>
<223>  Artificial Sequence


<220>
<221>  misc_feature
<222>  (1)..(22)
<223>  Sense primer of the universal primer set 1

<400>  14
gttatagggg taggaaagtg ag                                                22


<210>  15
<211>  23
<212>  DNA
<213>  Unknown

<220>
<223>  Artificial Sequence


<220>
<221>  misc_feature
<222>  (1)..(23)
<223>  Antisense primer of the universal primer set 1

<400>  15
ataaactcca tcttaatctt cac                                               23


<210>  16
<211>  22
<212>  DNA
<213>  Unknown

<220>
<223>  Artificial Sequence


<220>
<221>  misc_feature
<222>  (1)..(22)
<223>  Sense primer of the universal primer set 2

<400>  16
atgaatttta ttttgatttt ta                                                22


<210>  17
<211>  21
<212>  DNA
<213>  Unknown

<220>
<223>  Artificial Sequence


<220>

<221>  misc_feature
<222>  (1)..(21)
<223>  Antisense primer of the universal primer set 2

<400>  17
ccacaaaaac aaaaaaataa a                                                21


<210>  18
<211>  24
<212>  DNA
<213>  Unknown

<220>
<223>  Artificial Sequence


<220>
<221>  misc_feature
<222>  (1)..(24)
<223>  Sense primer of the cytosine methylation-specific primer set

<400>  18
ggtattagta gtagcgggag tgtc                                             24


<210>  19
<211>  24
<212>  DNA
<213>  Unknown

<220>
<223>  Artificial Sequence


<220>
<221>  misc_feature
<222>  (1)..(24)
<223>  Antisense primer of the cytosine methylation-specific primer set

<400>  19
ccaacttaaa tcgataacat aacg                                             24


<210>  20
<211>  25
<212>  DNA
<213>  Unknown

<220>
<223>  Artificial Sequence


<220>
<221>  misc_feature
<222>  (1)..(25)
<223>  Sense primer of the unmethylation-specific primer set

<400>  20
gtattagtag tagtgggagt gttgg                                            25


<210>  21
<211>  26
<212>  DNA

<213>  Unknown

<220>
<223>  Artificial Sequence



<220>
<221>  misc_feature
<222>  (1)..(26)
<223>  Antisense primer of the unmethylation-specific primer set

<400>  21
ccccaactta aatcaataac ataaca                                          26

## Description

### TECHNICAL FIELD OF THE INVENTION

The present invention relates to the methods of predicting the ability of malignant tumor invasion and metastasis in vitro, and also relates to the nucleotide fragments used in said methods.

### BACKGROUND OF THE INVENTION

Invasion and metastasis are predominantly reasons for the poor prognosis of cancers. Destructions of the neighbor and distant organs by cancer invasion and metastasis lead to loss of chance for surgical resection and recurrence after curative treatments. Sensitive biomarkers for detection of potential of invasion and metastasis would greatly improve the personalized clinical management for cancer patients. Therefore, predicting the invasion and metastasis potential of cancers is eagerly awaited.

It is well recognized that it is virtually impossible to identify metastasis potential of cancers based on histopathologic grounds alone. The great progressions have been achieved on molecular biology and carcinogenesis in the past three decades. It is expected these progressions should efficiently improve the development of personalized medicine. Although some protein or mRNA candidates (including MMP7) have been reported as predictors of prognosis of cancers, however, a practical molecular invasion or metastasis biomarker is still not available for clinical practice.

One of the remarkable recent discoveries on molecular carcinogenesis is epigenetic alteration of tumor related genes. In addition to the genetic inactivation of tumor suppressor genes (including p53 and APC), epigenetic inactivation of tumor suppressor genes (including p15, p16, hMLH1) by methylation of CpG islands is another kinds of frequently events in cancers. It is well known that detection of alterations of total proteins and total mRNA of gene expression in a few cells in tissues is very difficult with regular gene expression assays, because their visibility would be greatly reduced by the co-existent main cell populations in which the gene expression has not changed. In contrast, methylated and demethylated CpG islands can be analyzed with methylation- and demethylation-specific assays, respectively. This makes the detection of the methylation status of CpG islands so sensitive that methylation alterations that occurred in a few cells from a particular tissue can be clearly displayed. This makes DNA methylation an optimal biomarker for molecular stratification of cancers.

Serum response factor (SRF) is a master regulator of cell proliferation, differentiation, migration in various types of cells and tumor stroma (Medjkane S, et al. Myocardin-related transcription factors and SRF are required for cytoskeletal dynamics and experimental metastasis. Nat Cell Biol 2009, 11:257-268). As a metastasis-promoting gene, SRF is correlated with invasion and metastasis of cancers in the liver, pancreas, colorectal, thyroid, and other organs. However, it has not been reported that SRF can be inactivated by methylation of CpG islands and that inactivation SRF by methylation can be used to predict loss of invasion and metastasis potential of cancers.

### DETAILED DESCRIPTION OF THE INVENTION

Among various current techniques, no assay can be used to detect potential of invasion and metastasis of cancer for clinical practice. The present invention provides for an in vitro detection assay for invasion and metastasis potential of cancers by determination of cytosine methylation of 5' CpG islands around the transcription start site of the SRF gene in genomic DNA from tissue samples.

On one hand, the present invention provides for an in vitro detection assay for invasion and metastasis potential of cancers, which including the following three steps.
a) Extraction of genomic DNA from surgical margin or biopsy sample around cancer lesion;
b) Detection of cytosine methylation of 5' CpG islands around the transcription start site of the SRF gene DNA sequence in the DNA sample prepared in the above step a);
c) When cytosine methylation of SRF 5' CpG islands is observed in the step b), the tested cancer is highly resistant to cancer invasion and metastasis.

On another hand, the present invention further provides four artificial DNA sequences. One sequence contains a sequence fragment as listed on SEQ ID NO:5, or SEQ ID NO: 6. Another sequence contains a sequence fragment as listed on SEQ ID NO:7, or SEQ ID NO:8. Another sequence contains a sequence fragment as listed on SEQ ID NO:9, or SEQ ID NO:10. Another sequence contains a sequence fragment as listed on SEQ ID NO:11, or SEQ ID NO:12.which are used to design MSP primers for detection of SRF methylation.

Together, the inventors in the present invention find for the first time that invasion and metastasis potential of cancers can efficiently and precisely be determined through analysis of the methylation status of SRF 5' CpG islands in tissue sample. The inventors in the present invention also provide four artificial DNA sequences that are used to detect of the cytosine methylation status of SRF CpG islands.

### BRIEF DESCRIPTION OF THE FIGURES

Figure 1: (**A**) Illustration of SRF CpG island; (**B**) Ratio of cytosine methylation signal at each array probe within SRF CpG island in gastric carcinoma (GC) to its corresponding surgical margin (SM); (**C**) DHPLC chromatogram of PCR products of the SRF exon-1 region with and without cytosine methylation.
Figure 2: Kaplan-Meier analysis of post-operational overall survival (months) of gastric cancer patients with or without methylation of SRF CpG islands in their surgical margin samples.
Figure 3: Results of bisulfite sequencing show locations of the methylated CpG sites within SRF CpG islands in gastric samples. Samples F0859 and F0860 are SRF methylation-positive; Sample F0974 is SRF methylation-negative; each line represents one clone; the open circles represent unmethylated-CpG site; the filled circles represent the methylated-CpG sites; The regions marked with panes show the crucial CpG sites, the left marked region is corresponding to those crucial CpG sites from the 86^{th} to the 161^{st} nucleotide residues on the SEQ ID NO:1.
Figure 4: DHPLC chromatogram of PCR products of the SRF exon-1 region with and without cytosine methylation in tissues around thyroid cancer.
Figure 5: DHPLC chromatogram of PCR products of the SRF exon-1 region with and without cytosine methylation in tissues around oral cancer.

### DETAILED DESCRIPTIONS OF THE METHODS

SRF is an important proto-oncogene that contains a CpG island around its transcription start site and promoter. The methylation status of this CpG island controls the possibility of SRF transcription. The inventors in the present invention have found that SRF gene is not methylated and transcribed weakly in normal tissues. However, expression of SRF is upregulated in cancers. The inventors in this invention hypothesis that epigenetic inactivation of SRF by methylation in host stromal cells in cancer and its surround tissues in the target organ may inhibit invasion and metastasis potential of cancer cells, and that determination of SRF methylation in the tissue surrounding cancer may be an useful method for prediction of host defending capacity against cancer invasion and metastasis.

To approve above hypothesis, the inventors in the present invention have designed and carried out a set of experiments listed below. Results of these studies demonstrate that their hypothesis is true and can be used clinically. These experiments include following three basic steps.
1. Extraction of genomic DNA. DNA samples (>10ng per sample) are respectively extracted from surgical margin tissue or endoscopic biopsy surrounding cancer, positive control cells with SRF methylation, and negative control cells without SRF methylation;
2. Detection of cytosine methylation of SRF CpG islands. The unmethylated cytosine residues in genomic DNA are converted to uracil residues by treatments of chemicals such as bisulfite. Then, based on the bisulfite-modified sequence of SRF CpG islands, the CpG-free universal primer set is designed, synthesized, and used to amplify both methylated and unmethylated SRF CpG islands. [Alternatively, methylation-specific primer set matched to the methylated SRF CpG islands can also be designed and used to detect SRF methylation with methylation-specific PCR (MSP) or quantitative MSP (MethyLight).] The methylation status of SRF CpG islands is determined through analysis these PCR products by denatured high performance liquid chromatography (DHPLC), clone-sequencing, or other available assays.
3. Prediction of invasion and metastasis of cancer using SRF methylation as the biomarker.

### Validation Experimental-1.

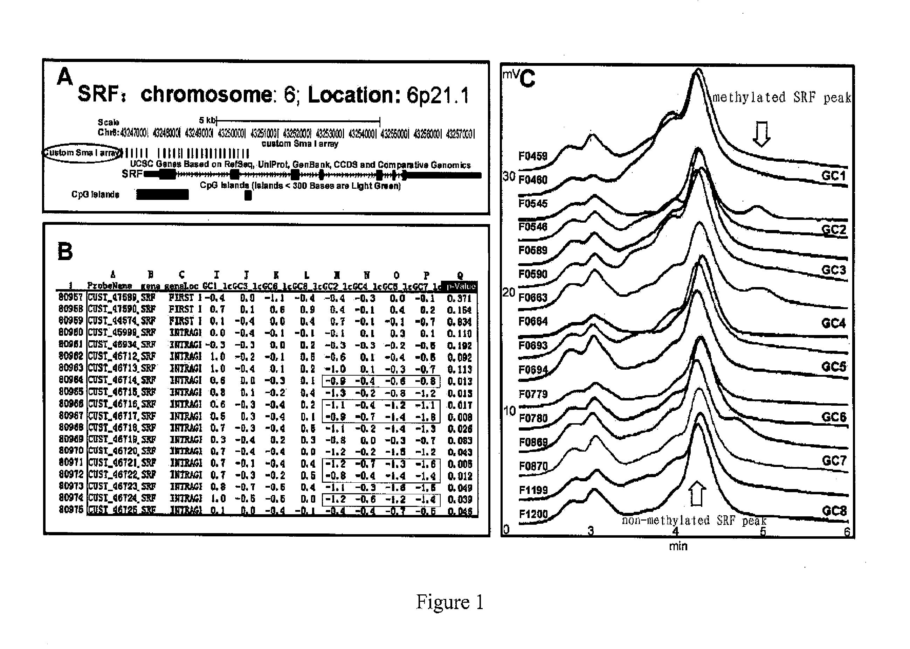

Using their methylation array data, the inventors in the present invention have found that the prevalence of SRF cytosine methylation in the surgical margin samples from patients with non-metastatic gastric carcinoma is significantly higher than that from patients with metastatic gastric carcinoma. Similar results are obtained in DHPLC analysis (Figure 1).

### Validation Experimental-2.

Using the DHPLC assay, the inventors in the present invention have detected SRF cytosine methylation in the surgical margin samples from patients with thyroid cancers.

### Validation Experimental-3.

Using the DHPLC assay, the inventors in the present invention have detected SRF cytosine methylation in the surgical margin samples from patients with oral cancers.

### Validation Experimental-4.

Using the DHPLC assay, the inventors in the present invention have detected SRF cytosine methylation in the surgical margin samples from patients with liver cancers.

### Validation Experimental-5.

Using the DHPLC assay, the inventors in the present invention have detected SRF cytosine methylation in the surgical margin samples from patients with breast cancers.

### Validation Experimental-6.

Using the DHPLC assay, the inventors in the present invention have detected SRF cytosine methylation in the surgical margin samples from patients with colorectal cancers.

### Validation Experimental-7.

Using the DHPLC assay, the inventors in the present invention have detected SRF cytosine methylation in the surgical margin samples from patients with pancreatic cancers.

### Validation Experimental-8.

Using the DHPLC assay, the inventors in the present invention have detected SRF cytosine methylation in the surgical margin samples from patients with lung cancers.

According to the results of above experiments, the inventors in the present invention provide an in vitro method to predict the ability of cancer invasion and metastasis. The novel method comprises three major steps:
a) Extraction of genomic DNA from surgical margin or biopsy sample around cancer;
b) Detection of the cytosine methylation status of SRF 5' CpG islands in the DNA prepared in the step a);
c) When SRF cytosine methylation is determined in the step b), it indicates that the tested tissue is highly resistant to cancer invasion and metastasis.

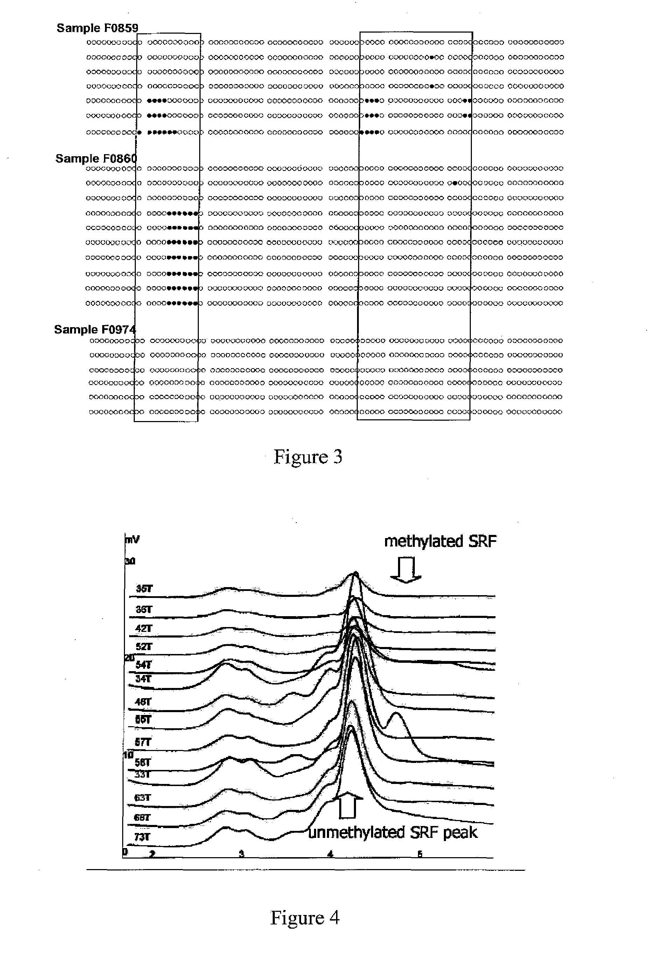

All the persons working on these fields understand that DNA sample in the step a) can be prepared by any methods that can be used for extraction of genomic DNA, including regular phenol: chloroform protocol and various DNA extraction kits from commercial companies such as Qiagen. When methylated CpG sites are detected in the SRF CpG islands in the step b), it is indicated the methylated SRF-containing tissue is highly resistant to cancer invasion and metastasis. For example, as illustrated in the Figure 3, when a methylated-CpG containing SRF clone is detected in a sample, this sample is at the low risk of invasion and metastasis.

As an optimized method to increase the detection accuracy, a assay is further developed and used in the step b) for detection of cytosine methylation of SRF 5' CpG islands at the crucial CpG sites from the 86^{th} to the 161^{st} nucleotides or from the 367^{th} to the 505^{st} nucleotides in the DNA sample prepared in the step a).

The sequence corresponding to the 5' fragment of the SRF gene mentioned above is illustrated as SEQ ID NO:13. In detail, the crucial CpG sites in SRF CpG islands are underlined in the following sequence.

5'-GCCACAGGGGCAGGAAAGTGAGGGCCCAGGTCGGCCCGGGCGTGCAGGGGCCCC
GGGTTCGCAGCGGCGGCCGCGGCAGCGATAGCGGCACTAGCAGCAGCGGGAGTGCC
GGGTTGAGCCGGGAAGCCGATGGCGGCGGCTGCGGCGGCTCCGATTCCTCGCTGACT
GCCCGTCCGCCCTCCTGCATCGAGCGCCATGTTACCGACCCAAGCTGGGGCCGCGGC
GGCTCTGGGCCGGGGCTCGGCCCTGGGGGGCAGCCTGAACCGGACCCCGACGGGGC
GGCCGGGCGGCGGCGGCGGGACACGCGGGGCTAACGGGGGCCGGGTCCCCGGGAA
TGGCGCGGGGCTCGGGCCCGGCCGCCTGGAGCGGGAGGCTGCGGCAGCGGCGGCA
ACCACCCCGGCGCCCACCGCGGGGGCCCTCTACAGCGGCAGCGAGGGCGACTCGGA
GTCGGGCGAGGAGGAGGAGCTGGGCGCCGAGCGGCGCGGCCTGAAGCGGAGCCTGA
GCGAGATGGAGATCGGTATGGTGGTCGGTGGGCCCGAGGCGTCGGCAGCGGCCACCG
GGGGCTACGGGCCGGTGAGCGGCGCGGTGAGCGGGGCCAAGCCGGGTAAGAAGACC

CGGGGCCGCGTGAAGATCAAGATGGAGTTCAT-3'

All the persons working on these fields understand that cytosine methylation of SRF 5' CpG islands described in the step b) can be detected by any methods that can be used for analysis of DNA methylation. As an optimized method to increase the accuracy for detection of SRF methylation, the detailed processes used in the step b) are listed below.
1) The unmethylated cytosine residues in genomic DNA samples prepared in the step a) are converted to uracil residues by treatments of chemicals, whereas the methylated cytosine residues are not converted. Therefore, the treated DNA mixtures contain both DNA molecules with methylated cytosine residues and DNA molecules without cytosine residues.
2) Primer set is designed according to the sequence SEQ ID NO:1 or SEQ ID NO:3, and used to amplify the corresponding amplicons using the converted DNA prepared in the step 1) as the templates.
3) Determination of cytosine methylation of SRF 5' CpG islands in the PCR products prepared in the step 2) using DHPLC or clone sequencing.

An alternatively method to detect cytosine methylation of SRF 5' CpG islands described in the step b) is also listed below.
1) Fragment the DNA samples prepared in the step a) by ultrasonic treatment or digestion with restriction enzymes. The average size of the resulted DNA fragments is about 200 base pairs.
2) Enrichment of DNA molecules containing methylated cytosine residues from the DNA fragments prepared in the step 1) using methylated DNA binding proteins or 5-methylcytosine-specific antibody.
3) Carry out PCR amplification using the enriched DNA molecules prepared in the step 2) as templates, and using the primer set specific to cytosine methylation and designed based on SEQ ID NO:1 or SEQ ID NO. 3. When the SRF PCR products are amplified successfully, it indicates that SRF cytosine methylation is detected in the tested DNA samples. OR directly sequencing DNA molecules prepared in the step 2), when the cytosine methylation of SRF 5'CpG islands is detected, it indicates that SRF cytosine methylation is detected in the tested DNA samples; OR label the enriched DNA prepared in the step 2) and incubate the labeled DNA molecules with the SRF 5' CpG island-specific probes for hybridization; When hybridization between the labeled DNA molecules and the SRF-specific probes is observed, it indicates that SRF cytosine methylation is detected in the tested DNA samples.

Another alternatively method to detect cytosine methylation of SRF 5' CpG islands described in the step b) is also listed below.
1) Digest the DNA sample prepared in the step a) with methylation-sensitive restriction enzymes.
2) Incubate the digested DNA prepared in the step 1) with the labeled SRF 5' CpG island-specific probes.
3) When hybridization between the digested DNA molecules and the labeled SRF-specific probes is observed, it indicates that SRF cytosine methylation is detected in the tested DNA samples.

Although any primer sets designed according to the mentioned SRF-related sequences can be used to detect the methylation status of SRF CpG islands, as the primer set used in an optimized method to increase the detection accuracy for SRF methylation, the primer set can be designed according to the sequence SEQ ID NO.1 or SEQ ID NO.3. The universal primer set can be consisted of the sense primer 5'-gttataggggtaggaaagtgag-3' and the antisense primer 5'-ataaactccatcttaatcttcac-3', or the sense primer 5'-atgaattttattttgattttta-3' and the antisense primer 5'-ccacaaaaacaaaaaaataaa-3'.

As primer sets used to detect the methylation status of SRF CpG islands in an alternative method, these primer sets can also be cytosine methylation-specific primer sets designed according to the sequence SEQ ID NO.1 or SEQ ID NO.3. The cytosine methylation-specific primer set can be consisted of the sense primer 5'-ggtattagtagtagcgggagtgtc-3' and the antisense primer 5'-ccaacttaaatcgataacataacg-3'. To avoid the result of negative failure in detection of methylated SRF using cytosine methylation-specific primer set, an unmethylation-specific primer set can be designed according to the unmethylated sequences of SRF CpG islands in which the unmethylated cytosine residues are converted to uracil residues after chemical modification, and used to confirm that the negative detection of SRF methylation is because of no CpG methylation in SRF CpG islands, but not the absence of DNA templates. The unmethylation-specific primer set can be consisted of the sense primer 5'-gtattagtagtagtgggagtgttgg-3' and the antisense primer 5'-ccccaacttaaatcaataacataaca -3'.

As another optimized method to increase the detection accuracy, when the number of cytosine methylated 5' CpG sites in a clone of SRF CpG islands detected in the step b) is equal to or more than 4, it indicates that the tested tissue sample is resistant to cancer invasion and metastasis; or when the number of cytosine methylated 5' CpG sites in a clone of SRF CpG islands detected in the step b) is equal to or more than 6, it indicates that the tested tissue sample is highly resistant to cancer invasion and metastasis.

Alternatively, when the number of cytosine methylated 5' CpG sites from the nucleotide 86^{th} to the nucleotide 161^{st} in a clone of SRF 5' CpG islands detected in the step b) is equal to or more than 4, it indicates that the tested tissue sample is resistant to cancer invasion and metastasis; or when the number of cytosine methylated 5' CpG sites from the nucleotide 367^{th} to the nucleotide 505^{th} in a clone of SRF CpG islands detected in the step b) is equal to or more than 4, it indicates that the tested tissue sample is resistant to cancer invasion and metastasis.

Although cancers described in this invention can be any kinds of malignant tumors in which SRF is expressed, as the cancers analyzed with an optimized method, the cancers described include cancers in the stomach, thyroid, oral cavity, liver, breast, lung, pancreas, column, etc.

The SEQ ID NO:1 shows the DNA sequence of the SENSE strand of SRF 5' CpG island in which all cytosine residues in CpG sites are methylated and all cytosine residues in non-CpG sites are chemically converted to uracil residues. This sequence can be used to design methods for detection of the methylation status of SRF 5' CpG islands. In detail, this sequence is used as the standard template sequence to design the PCR primer sets used to detect cytosine methylated SRF 5' CpG islands.

The SEQ ID NO:2 shows the DNA sequence of the SENSE strand of SRF 5'CpG island in which all cytosine residues are chemically converted to uracil residues. This sequence can be used to design methods for detection of the methylation status of SRF 5' CpG islands. In detail, this sequence is used as the standard template sequence to design the primer sets used to detect the unmethylated SRF 5' CpG islands, which are used to confirm negative detection of SRF methylation.

The SEQ ID NO:3 shows the DNA sequence of the ANTISENSE strand of SRF 5' CpG island in which all cytosine residues in CpG sites are methylated and all cytosine residues in non-CpG sites are chemically converted to uracil residues. The usages of this sequence are the same as the sequence SEQ ID NO:1.

The SEQ ID NO:4 shows the DNA sequence of the ANTISENSE strand of SRF 5' CpG island in which all cytosine residues are chemically converted to uracil residues. The usages of this sequence are the same as the sequence SEQ ID NO:2.

Four sequences SEQ ID NO:1-4 described above are listed below in detail.

The SEQ ID NO:1 is listed below (the underline sequences are two crucial CpG methylation regions; the paned sequences are matched to the universal primer set).

5'-GUUAUAGGGGUAGGAAAGTGAGGGUUUAGGTCGGUUCGGGCGTGUAGGGGU
UUCGGGTTCGUAGCGGCGGUCGCGGUAGCGATAGCGGUAUTAGUAGUAGCGGGAGT
GUCGGGTTGAGUCGGGAAGUCGATGGCGGCGGUTGCGGCGGUTCGATTUUTCGUTG
AUTGUUCGTUCGUUUTUUTGUATCGAGCGUUATGTTAUCGAUUUAAGUTGGGGUCGCG
GCGGUTUTGGGUCGGGGUTCGGUUUTGGGGGUAGUUTGAAUCGGAUUUCGACGGG
GCGGUCGGGCGGCGGCGGCGGGAUACGCGGGGUTAACGGGGGUCGGGTUUUCGGG
AATGGCGCGGGGUTCGGGUUCGGUCGUUTGGAGCGGGAGGUTGCGGUAGCGGCGG
UAAUUAUUUCGGCGUUUAUCGCGGGGGUUUTUTAUAGCGGUAGCGAGGGCGAUTCG
GAGTCGGGCGAGGAGGAGGAGUTGGGCGUCGAGCGGCGCGGUUTGAAGCGGAGUUT
GAGCGAGATGGAGATCGGTATGGTGGTCGGTGGGUUCGAGGCGTCGGUAGCGGUUAU
CGGGGGUTACGGGUCGGTGAGCGGCGCGGTGAGCGGGGUUAAGUCGGGTAAGAAGA
UUCGGGGUCGCGTGAAGATUAAGATGGAGTTUAT-3'

The SEQ ID NO:2 is listed below. All the cytosine residues in the SEQ ID NO:1 are converted to uracil residues because they are not methylated (the underline sequences are two crucial regions in which all cytosine residues that are not methylated are chemically converted to uracil residues; the paned sequences are matched to the universal primer set).

5'-GUUAUAGGGUAGGAAAGTGAGGGUUUAGGTUGGUUUGGGUGTGUAGGGGUU
UUGGGTTUGUAGUGGUGGUUGUGGUAGUGATAGUGGUAUTAGUAGUAGUGGGAGTG

UUGGGTTGAGUUGGAAGUUGATGGUGGUGGUTGUGGUGGUTUUGATTUUTUGUTGA
UTGUUUGTUUGUUUTUUTGUATUGAGUGUUATGTTAUUGAUUUAAGUTGGGUUGUGG
UGGUTUTGGGUUGGGGUTUGGUUUTGGGGGUAGUUTGAAUUGGAUUUUGAUGGGG
UGGUUGGGUGGUGGUGGUGGGAUAUGUGGGGUTAAUGGGGUUGGGTUUUUGGGA
ATGGUGUGGGGUTUGGGUUUGGUUGUUTGGAGUGGGAGGUTGUGGUAGUGGUGGU
AAUUAUUUUGGUGUUUAUUGUGGGGGUUUTUTAUAGUGGUAGUGAGGGUGAUTUGG
AGTUGGGUGAGGAGGAGGAGUTGGGUGUUGAGUGGUGUGGUUTGAAGUGGAGUUTG
AGUGAGATGGAGATUGGTATGGTGGTUGGTGGGUUUGAGGUGTUGGUAGUGGUUAUU
GGGGGUTAUGGGUUGGTGAGUGGUGUGGTGAGUGGGGUUAAGUUGGGTAAGAAGAU
UUGGGGUUGUGTGAAGATUAAGATGGAGTTUAT-3'

The SEQ ID NO:3 is listed below (the underline sequences are two crucial CpG methylation regions).

5'-ATGAAUTUUATUTTGATUTTUACGCGGUUUCGGGTUTTUTTAUUCGGUTTGGUUU
CGUTUAUCGCGUCGUTUAUCGGUUCGTAGUUUUCGGTGGUCGUTGUCGACGUUTCGG
GUUUAUCGAUUAUUATAUCGATUTUUATUTCGUTUAGGUTUCGUTTUAGGUCGCGUCG
UTCGGCGUUUAGUTUUTUUTUUTCGUUCGAUTUCGAGTCGUUUTCGUTGUCGUTGTAG
AGGGUUUUCGCGGTGGGCGUCGGGGTGGTTGUCGUCGUTGUCGUAGUUTUUCGUTU
UAGGCGGUCGGGUUCGAGUUUCGCGUUATTUUCGGGGAUUCGGUUUUCGTTAGUUU
CGCGTGTUUCGUCGUCGUCGUUCGGUCGUUUCGTCGGGGTUCGGTTUAGGUTGUUU
UUUAGGGUCGAGUUUCGGUUUAGAGUCGUCGCGGUUUUAGUTTGGGTCGGTAAUATG
GCGUTCGATGUAGGAGGGCGGACGGGUAGTUAGCGAGGAATCGGAGUCGUCGUAGU
CGUCGUUATCGGUTTUUCGGUTUAAUUCGGUAUTUUCGUTGUTGUTAGTGUCGUTATC
GUTGUCGCGGUCGUCGUTGCGAAUUCGGGGUUUUTGUACGUUCGGGUCGAUUTGGG
UUUTUAUTTTUUTGUUUUTGTGG-3'

The SEQ ID NO:4 is listed below. All the cytosine residues in the SEQ ID NO:3 are converted to uracil residues because they are not methylated (the underline sequences are two crucial regions in which all cytosine residues that are not methylated are chemically converted to uracil residues).

5’-ATGAAUTUUATUTTGATUTTUAUGUGGUUUUGGGTUTTUTTAUUUGGUTTGGUUU
UGUTUAUUGUGUUGUTUAUUGGUUUGTAGUUUUGGTGGUUGUTGUUGAUGUUTUGG

GUUUAUUGAUUAUUATAUUGATUTUUATUTUGUTUAGGUTUUGUTTUAGGUUGUGUUG
UTUGGUGUUUAGUTUUTUUTUUTGUUUGAUTUUGAGTUGUUTUGUTGUUGUTGTAG
AGGGUUUUUGUGGTGGGUGUUGGGTGGTTGUUGUUGUTGUUGUAGUUTUUUGUTU
UAGGUGGUUGGGUUUGAGUUUUGUGUUATTUUUGGGGAUUUGGUUUUUGTTAGUUU
UGUGTGTUUUGUUGUUGUUGUUUGGUUGUUUUGTUGGGGTUUGGTTUAGGUTGUUU
UUUAGGGUUGAGUUUUGGUUUAGAGUUGUUGUGGUUUUAGUTTGGGTUGGTAAUATG
GUGUTUGATGUAGGAGGGUGGAUGGGUAGTUAGUGAGGAATUGGAGUUGUUGUAGU
UGUUGUUATUGGUTTUUUGGUTUAAUUUGGUAUTUUUGUTGUTGUTAGTGUUGUTATU
GUTGUUGUGGUUGUUGUTGUGAAUUUGGGGUUUUTGUAUGUUUGGGUUGAUUTGGG
UUUTUAUTTTUUTGUUUUTGTGG-3’

The inventors in the present invention also provide an artificial nucleotide oligo listed as the SEQ ID NO:5. This oligo is the same as the 5'-underlined crucial sequence in the SEQ ID NO:1. The usages of SEQ ID NO:5 are similar to those of the SEQ ID NO:1. However, the detection accuracy of the methods designed for analysis of SRF methylation according to the SEQ ID NO:5 is higher than that of the methods designed according to the SEQ ID NO:1.

The inventors in the present invention also provide an artificial nucleotide oligo listed as the SEQ ID NO:6. This oligo is the same as the 5'-underlined crucial sequence in the SEQ ID NO:2. The usages of SEQ ID NO:6 are similar to those of the SEQ ID NO:2. However, the detection accuracy of the methods designed for analysis of SRF methylation according to the SEQ ID NO:6 is higher than that of the methods designed according to the SEQ ID NO:2.

The inventors in the present invention also provide an artificial nucleotide oligo listed as the SEQ ID NO:7. This oligo is the same as the 3'-underlined crucial sequence in the SEQ ID NO:3. The usages of SEQ ID NO:7 are the same as to those of the SEQ ID NO:5.

The inventors in the present invention also provide an artificial nucleotide oligo listed as the SEQ ID NO:8. This oligo is the same as the 3'-underlined crucial sequence in the SEQ ID NO:4. The usages of SEQ ID NO:8 are the same as to those of the SEQ ID NO:6.

The inventors in the present invention also provide an artificial nucleotide oligo listed as the SEQ ID NO:9. This oligo is the same as the 3'-underlined crucial sequence in the SEQ ID NO:1. The usages of SEQ ID NO:9 are the same as to those of the SEQ ID NO:5.

The inventors in the present invention also provide an artificial nucleotide oligo listed as the SEQ ID NO:10. This oligo is the same as the 3'-underlined crucial sequence in the SEQ ID NO:2. The usages of SEQ ID NO:10 are the same as to those of the SEQ ID NO:6.

The inventors in the present invention also provide an artificial nucleotide oligo listed as the SEQ ID NO:11. This oligo is the same as the 5'-underlined crucial sequence in the SEQ ID NO:3. The usages of SEQ ID NO:11 are the same as to those of the SEQ ID NO:5.

The inventors in the present invention also provide an artificial nucleotide oligo listed as the SEQ ID NO:12. This oligo is the same as the 5'-underlined crucial sequence in the SEQ ID NO:4. The usages of SEQ ID NO:12 are the same as to those of the SEQ ID NO:6.

The sequences of SEQ ID NO:5-12 are listed below.

The SEQ ID NO:5 has the following sequence,

5’-CGGUAUTAGUAGUAGCGGGAGTGUCGGGTTGAGUCGGGAAGUCGATGGCGGC
GGUTGCGGCGGUTUCGATTUUTC-3’

The SEQ ID NO:6 has the following sequence,

5’-UGGUAUTAGUAGUAGUGGGAGTGUUGGGTTGAGUUGGGAAGUUGATGGUGGU
GGUTGUGGUGGUTUUGATTUUTGU-3’

The SEQ ID NO:7 has the following sequence,

5'-CGAGGAATCGGAGUCGUCGUAGUCGUCGUUATCGGUTTUUCGGUTUAAUUCGG
UAUTUUCGUTGUTGUTAGTGUCG-3'

The SEQ ID NO:8 has the following sequence,

5’-UGAGGAATUGGAGUUGUUGUAGUUGUUGUUATUGGUTTUUUGGUTUAAUUUGG
UAUTUUUGUTGUTGUTAGTGUUG-3’

The SEQ ID NO:9 has the following sequence,

5’-CGGGAGGUTGCGGUAGCGGCGGUAAUUAUUUCGGCGUUUAUCGCGGGGUU
UTUTAUAGCGGUAGCGAGGGCGAUTCGGAGTCGGGCGAGGAGGAGGAGUTGGGCGU
CGAGCGGCGCGGUUTGAAGCGGAGUUTGAGCG-3’

The SEQ ID NO:10 has the following sequence,

5'-UGGGAGGUTGUGGUAGUGGUGGUAAUUAUUUUGGUGUUUAUUGUGGGGUU
UTUTAUAGUGGUAGUGAGGGUGAUTUGGAGTUGGGUGAGGAGGAGUTGGGUGU
UGAGUGGUGUGGUUTGAAGUGGAGUUTGAGUG-3'

The SEQ ID NO:11 has the following sequence,

5’-CGUTUAGGUTUCGUTTUAGGUCGCGUCGUTCGGCGUUUAGUTUUTUUTUUTCG
UUCGAUTUCGAGTCGUUUTCGUTGUCGUTGTAGAGGGUUUUCGCGGTGGGCGUCGG
GGTGGTTGUCGUCGUTGUCGUAGUUTUUCG-3’

The SEQ ID NO:12 has the following sequence,

5’-UGUTUAGGUTUUGUTTUAGGUUGUGUUGUTUGGUGUUUAGUTUUTUUTUUTUG
UUUGAUTUUGAGTUGUUUTUGUTGUUGUTGTAGAGGGUUUUGUGGTGGGUGUUGG
GGTGGTTGUUGUUGUTGUUGUAGUUTUUUG-3’

### EXAMPLES

### Example-1: Prediction of invasion and metastasis of gastric carcinomas by determination of cytosine methylation of SRF CpG islands in the surgical margin tissues from the cancer patients

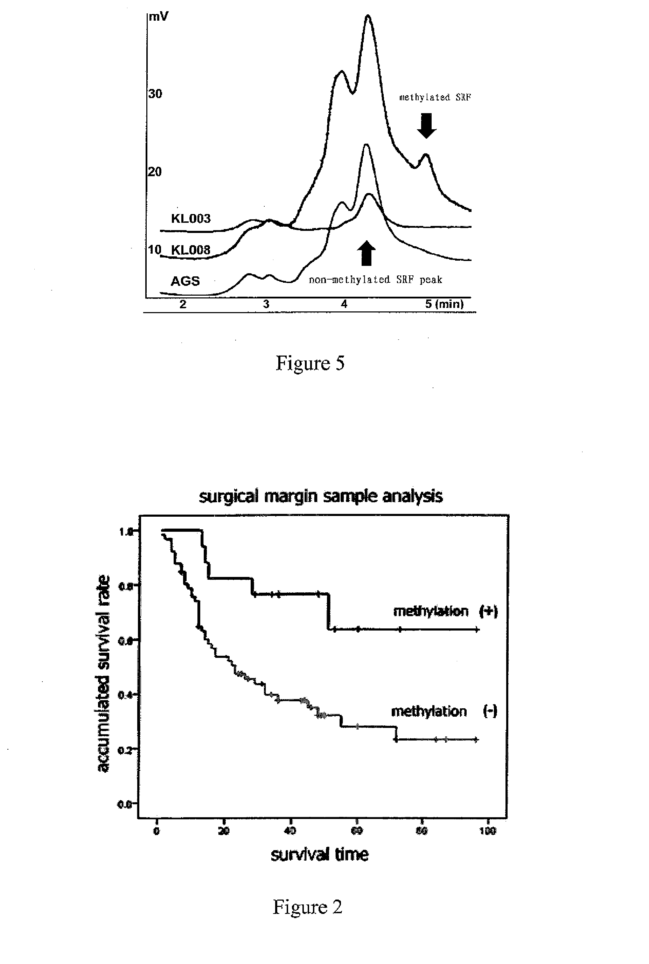

1. Subjects: Frozen fresh gastric carcinomas and surgical margin samples from 102 patients. Half of these gastric carcinomas are metastatic and vessel embolus-positive; the rest half of these carcinomas are non-metastatic and vessel embolus-negative. The clinicopathological and followup information are available for each patient.
2. Tissue sampling: Take 5 pieces (15-20 mm³) of fresh gastric samples at different sites along the surgical cutting edges of the resected cancer and its surrounding tissue. Put these samples into a silicated microcentrifuge tube (1.5 ml).
3. DNA extraction: The tissue samples in the microcentrifuge are mixed with 300 µl of lysis buffer (10 mM Tris.Cl/pH7.6, 10 mM NaCl, 10 mM EDTA, and 0.5% SDS) and 10 µl of proteinase K solution (20 mg/ml), digested at 37°C overnight. Genomic DNA (about 10 µg) was extracted with typical phenol: chloroform protocol.
4. Chemical modification of unmethylated cytosine residues in DNA:
   1) Add 18 µl of distilled sterile water into 1.5 ml microcentrifuge tube added with 1 µg genomic DNA prepared in the step 3, and denatured in water bath at 95°C for 20 min.
   2) To denature double strands DNA, add 2 µl of 3M NaOH, mix, incubate at 42°C for 20 min.
   3) To sulfite DNA, add 380 µl of fresh 5.0 M Na₂S₂O₅ (equal to 3M NaHSO₃), mix. Cover the top of reaction with 200 µl of mineral oil to prevent evaporation of reaction. Incubate at 50°C∼55°C overnight (12h∼16h) to convert the unmethylated cytosine residues to uracil residues.
   4) Remove mineral oil. Purify the modified DNA with Wizard DNA Clean-Up System (Promega A7280) as the kit instruction: Attach syringe to minicolumn and insert the tip of column into the vacuum manifold, one set for one sample. Add 1 ml of resin solution into microcentrifuge tube, pipette resin/DNA mix down and up, transfer the mix into column-syringe assembly, remain 5 min. Apply a vacuum to draw the solution through the column. DNA-resin binding complex was remained on the column. Break the vacuum. To wash the complexes in the column, add 2 ml of 80% isopropanol to the syringe, and reapply a vacuum to draw the solution through the column. Dry the DNA-resin complexes by continuing to draw a vacuum for 30sec after the solution has been pulled through the column, transfer the column to a 1.5 ml microcentrifuge tube. Centrifuge the column at maximum speed (10,000g) for 20 sec to remove any residual isopropanol. Transfer the column to a new microcentrifuge tube. Apply 50 µl of pre-warmed (80°C) water and remain 15 min at 80°C. Centrifuge the column for 20 sec at maximum speed (10,000g) to elute the bound DNA. Reapply 50 µl of pre-warmed (80°C) water and remain 15 min at room temperature (RT). Centrifuge again. Remove and discard the column.
   5) To complete the modification, add 11 µl of 3M NaOH, mix, remain 15 min at 37°C.
   6) To precipitate DNA and remove NaOH, add 166 µl of 5M NaOAC and 750 µl of 100% cold ethanol, mix, store at -20°C for 4h. Centrifuge at 10,000g for 30 min. Discard solution. Add 200 µl of 80% cold ethanol to wash DNA. Centrifuge again. Discard solution.
   7) Resuspend the DNA in 60 µl of sterile water or TE buffer. Use immediately or store at -20°C.
5. Design PCR primer set: According to the modified sequence SEQ ID NO:1 or the SEQ ID NO:2, design and synthesize CpG-free universal primer set. The primer set designed according to the SEQ ID NO:1 is consisted of the sense primer 5'-gttataggggtaggaaagtgag-3' and antisense primer 5'-ataaactccatctta atcttcac-3'.
6. PCR amplification: Carry out hot-start PCR using the primer set listed in the step 5 and the modified DNA prepared in the step 4 as the templates. Thermal cycle conditions are 95°C for 15 min, followed by 40 cycles of 95°C for 30 sec → 58°C for 30 sec → 72°C for 1 min, and 72°C for 10 min finally.
7. Detection of methylated SRF by DHPLC: The PCR products for the methylated SRF amplified in the step 6 are separated from the unmethylated ones by DHPLC (Figure 1 C) and confirmed by clone-sequencing (Figure 3).
8. Results: 1) SRF methylation-positive rate is 4 of 51 gastric surgical margin samples from patients with metastatic gastric cancer, and 16 of 51 surgical margin samples from patients with non-metastatic gastric cancer (7.8% versus 31.4%, P=0.003). 2) SRF methylation-positive rate is 1 of 30 gastric surgical margin samples from patients with gastric cancer at the pathological invasion depth T₄, and 19 of 72 surgical margin samples from patients with gastric cancer at the pathological invasion depth T₁₋₃ (3.3% versus 26.4%, P=0.014). 3). The overall survival of SRF methylation-positive patients is significantly longer than that of SRF methylation-negative patients in univariate and multivariate analysis (P=0.006 and 0.040, respectively; Figure 2). The 3-yrs overall survival rate is 32 months (median) for the SRF methylation-positive patients, 23 months for the SRF methylation-negative patients.
9. Conclusion: Methylation of SRF CpG islands in gastric surgical margin sample can be used as a biomarker to predict the invasion and metastasis of gastric cancers.

### Example-2: Prediction of invasion and metastasis of thyroid carcinomas by determination of cytosine methylation of SRF CpG islands in the surgical margin tissues from the cancer patients

1. Subjects: Frozen fresh thyroid carcinomas and surgical margin samples from 20 patients. Half of these gastric carcinomas are metastatic and vessel embolus-positive (or distant metastatic foci); the rest half of these carcinomas are non-metastatic and vessel embolus-negative (or distant metastatic foci). The Clinicopathological and followup information are available for each patients.
The steps 2-7 are the same as the step 2-7 in the Example-1;
8. Results are the same as example 1.
9. Conclusion: Methylation of SRF CpG islands in gastric surgical margin sample can be used as a biomarker to predict the invasion and metastasis of thyroid cancers.

### Example-3: Prediction of invasion and metastasis of gastric carcinomas by determination of cytosine methylation of SRF CpG islands in the surgical margin tissues from the cancer patients

The steps 1-6 are the same as the step 1-6 in the Example-1;
7. A new criterion is used to define a sample SRF methylation-positive. That is, only these samples are defined as SRF methylation-positive when the number of methylated CpG sites in a clone of SRF PCR products amplified in the step 6 in the Example-1 is equal to or more than 6, in the clone sequencing assay in the step 7 in the Example-1;
8. Using the new criterion, the specificity of prediction of invasion and metastasis of gastric cancers is higher than that in the Example-1 (100%).

### Example-4: Prediction of invasion and metastasis of gastric carcinomas by determination of cytosine methylation of SRF CpG islands in the surgical margin tissues from the cancer patients

The steps 1-4 are the same as the step 1-4 in the Example-1;
5. Design and synthesize the primer set: the sense primer 5'-atgaattttattttgattttta-3' and the antisense primer 5'-ccacaaaaacaaaaaaataaa-3'.
6. PCR amplification: Carry out hot-start PCR using the primer set synthesized in the step 5 and using the DNA prepared in the step 4 as the templates. Thermal cycle conditions are 95°C for 15 min, followed by 40 cycles of 95°C for 30 sec → 54°C for 30 sec → 72°C for 1 min, and 72°C for 10 min finally.
7. Results and conclusion: the same as the Example-1.

### Example-5: Prediction of invasion and metastasis of gastric carcinomas by determination of cytosine methylation of SRF CpG islands in the surgical margin tissues from the cancer patients

The steps 1-4 are the same as the step 1-4 in the Example-1;
5. Design and synthesize the primer set: the sense primer 5'-ggtattagtagtagcgggagtgtc-3' and the antisense primer 5'-ccaacttaaatcgataacataacg-3'.
6. PCR amplification: Carry out hot-start PCR using the primer set synthesized in the step 5 and using the DNA prepared in the step 4 as the templates. Thermal cycle condition are 95°C for 15 min, followed by 40 cycles of 95°C for 30 sec → 57°C for 30 sec → 72°C for 1 min, and 72°C for 10 min finally.
7. Results and conclusion: the same as the Example-1.

### Example-6: Prediction of invasion and metastasis of oral carcinomas by determination of cytosine methylation of SRF CpG islands in the surgical margin tissues from the cancer patients

As described in example 1, SRF methylation is also detected in surgical margin samples from patients with oral cancer (Figure 5). The results are similar to the Example-1.

### Example-7: Prediction of invasion and metastasis of pancreas carcinomas by determination of cytosine methylation of SRF CpG islands in the surgical margin tissues from the cancer patients

As described in example 1, SRF methylation is also detected in surgical margin samples from patients with pancreas cancer. The results are similar to the Example-1.

### Example-8: Prediction of invasion and metastasis of colon carcinomas by determination of cytosine methylation of SRF CpG islands in the surgical margin tissues from the cancer patients

As described in example 1, SRF methylation is also detected in surgical margin samples from patients with colon cancer. The results are similar to the Example-1.

### Example-9: Prediction of invasion and metastasis of breast carcinomas by determination of cytosine methylation of SRF CpG islands in the surgical margin tissues from the cancer patients

As described in example 1, SRF methylation is also detected in surgical margin samples from patients with breast cancer. The results are similar to the Example-1.

### Example-10: Prediction of invasion and metastasis of liver carcinomas by determination of cytosine methylation of SRF CpG islands in the surgical margin tissues from the cancer patients

As described in example 1, SRF methylation is also detected in surgical margin samples from patients with liver cancer. The results are similar to the Example-1.

### Example-11: Prediction of invasion and metastasis of lung carcinomas by determination of cytosine methylation of SRF CpG islands in the surgical margin tissues from the cancer patients

As described in example 1, SRF methylation is also detected in surgical margin samples from patients with lung cancer. The results are similar to the Example-1.

### Example-12: Prediction of invasion and metastasis of gastric carcinomas by determination of cytosine methylation of SRF CpG islands in the surgical margin tissues from the cancer patients

The steps 1-6 are the same as the step 1-6 in the Example-1;
7. Detect methylation of crucial CpG sites in clones of the PCR products prepared in the step 6 by clone sequencing. The minimum number of sequenced clones is 10 for one tested sample.

The steps 8-9 are the same as the steps 8-9 in the Example-1.

## Claims

1. A method of predicting the ability of malignant tumor invasion and metastasis in vitro, **characterized in that** said method comprises the following steps:
a) Extraction of genomic DNA from surgical margin or biopsy sample around cancer lesion;
b) Detection of cytosine methylation of 5' CpG islands around the transcription start site of the SRF gene DNA sequence in the DNA sample prepared in the above step a);
c) When cytosine methylation of SRF 5' CpG islands is observed in the step b), it indicates that the tested cancer is highly resistant to cancer invasion and metastasis.

2. A method according to claim 1, wherein detection of cytosine methylation of SRF 5' CpG islands in the step b) is at these CpG sites from the 86^{th} nucleotide to the 161^{st} nucleotide within SRF 5' CpG islands; OR wherein detection of cytosine methylation of SRF 5' CpG islands in the step b) is at these CpG sites from the 367^{th} nucleotide to the 505^{th} nucleotide within SRF 5' CpG islands.

3. A method according to claim 1, wherein detection of cytosine methylation of SRF 5'CpG islands in the step b) is analyzed as described below:
1) The unmethylated cytosine residues in the DNA sample prepared in the step a) are converted to uracil residues by chemical treatment, modified DNA mixture contains DNA molecules with methylated cytosine residues and DNA molecules without unmethylated cytosine residues;
2) PCR amplification using the DNA mixture prepared in the step 1) as the templates and using the universal primer set designed according to the sequence SEQ ID NO:1 or SEQ ID NO:3;
3) Determination of cytosine methylation of SRF 5' CpG islands in the PCR products prepared in the step 2) using DHPLC or clone sequencing;
Or, detection of cytosine methylation of SRF 5' CpG islands in the step b) is analyzed as described below:
1) Fragment the DNA molecules prepared in the step a) into about 200bp by ultrasonic treatment or digestion with restriction enzyme;
2) Enrichment of DNA fragments containing methylated cytosine residues in the DNA prepared in the step 1) using methylated DNA binding protein or antibody against 5-methylcytosine;
3) Carry out PCR amplification using the enriched DNA molecules prepared in the step 2) as templates, and using the primer set specific to cytosine methylation and designed based on SEQ ID NO:1 or SEQ ID NO. 3, when the SRF PCR products are amplified successfully, it indicates that SRF cytosine methylation is detected in the tested DNA samples; OR directly sequencing DNA molecules prepared in the step 2), when the cytosine methylation of SRF 5' CpG islands is detected, it indicates that SRF cytosine methylation is detected in the tested DNA samples; OR label the enriched DNA prepared in the step 2) and incubate the labeled DNA molecules with the SRF 5' CpG island-specific probes for hybridization; When hybridization between the labeled DNA molecules and the SRF-specific probes is observed, it indicates that SRF cytosine methylation is detected in the tested DNA samples.
Or, detection of cytosine methylation of SRF 5' CpG islands in the step b) is analyzed as described below:
1) Digest the DNA sample prepared in the step a) with methylation-sensitive restriction enzymes;
2) Incubate the digested DNA prepared in the step 1) with the labeled SRF 5' CpG island-specific probes;
3) When hybridization between the digested DNA molecules and the labeled SRF-specific probes is observed, it indicates that SRF cytosine methylation is detected in the tested DNA samples.

4. A method according to claim 3, wherein the universal primer set designed according to the sequence SEQ ID NO:1 or SEQ ID NO:3. is consisted of the sense primer 5'-gttataggggtaggaaagtgag-3' and antisense primer 5'-ataaactccatcttaatcttcac-3'; OR wherein the universal primer set designed according to the sequence SEQ ID NO:1 or SEQ ID NO:3. is consisted of the sense primer 5'-atgaattttattttgattttta-3' and antisense primer 5'-ccacaaaaacaaaaaaataaa-3'.

5. A method according to claim 3, wherein the cytosine methylation-specific primer set designed according to the sequence SEQ ID NO:1 or SEQ ID NO:3. is consisted of the sense primer 5'-ggtattagtagtagcgggagtgt-3' and antisense primer 5'-ccaacttaaatcgataacataacg-3'.

6. A method according to claim 1, wherein it indicates that the tested tissue sample is resistant to cancer invasion and metastasis when the number of methylated CpG sites in a clone of SRF 5' CpG islands detected in the step b) is equal to or more than 4.

7. The artificial sequence listed as SEQ ID NO:5 and SEQ ID NO:6.

8. The artificial sequence listed as SEQ ID NO:7 and SEQ ID NO:8.

9. The artificial sequence listed as SEQ ID NO:9 and SEQ ID NO:10.

10. The artificial sequence listed as SEQ ID NO:11 and SEQ ID NO:12.
